(19) Europäisches Patentamt
European Patent Office
Office européen des brevets

(11) **EP 4 197 441 A1**

(12) **EUROPEAN PATENT APPLICATION**

(43) Date of publication:
**21.06.2023 Bulletin 2023/25**

(21) Application number: **22205921.4**

(22) Date of filing: **07.11.2022**

(51) International Patent Classification (IPC):
*A61B 5/271* (2021.01)    *A61B 5/252* (2021.01)
*A61B 5/254* (2021.01)    *A61B 5/256* (2021.01)
*A61B 5/259* (2021.01)    *A61B 5/00* (2006.01)
*A61B 5/266* (2021.01)    *H01R 3/00* (2006.01)
*H01R 4/00* (2006.01)

(52) Cooperative Patent Classification (CPC):
**A61B 5/271; A61B 5/252; A61B 5/254;**
**A61B 5/256; A61B 5/259; A61B 5/6823;**
**A61B 5/6824; A61B 5/6828;** A61B 5/266;
A61B 2562/0209; A61B 2562/043; A61B 2562/14;
A61B 2562/222

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **16.12.2021 US 202117553414**

(71) Applicant: **Alivecor, Inc.**
**Mountain View,  CA 94041 (US)**

(72) Inventors:
• **NEGI, Indira**
  **San Jose, CA (US)**
• **ALBERT, David E**
  **Oklahoma City, OK (US)**
• **SATCHWELL, Bruce**
  **Queensland, Australia (AU)**
• **BARNETT, Kim Norman**
  **Brisbane, Australia (AU)**
• **XUE, Joel Q**
  **Wauwatosa, WI (US)**

(74) Representative: **Bartle Read**
**Liverpool Science Park**
**131 Mount Pleasant**
**Liverpool L3 5TF (GB)**

(54) **TWELVE-LEAD ELECTROCARDIOGRAM USING ELECTRODES COUPLED BY A SINGLE CABLE**

(57)    Embodiments of the present disclosure provide an ECG monitoring device that comprises a housing cable comprising a plurality of signal cables positioned therein and a set of electrodes positioned along the housing cable. Each of the set of electrodes may contact a particular location of a user without requiring any muscular activity of the user when the ECG monitoring device is connected to the user. The ECG monitoring device may further comprise a computing device positioned along the housing cable and operatively coupled to each of the four or more electrodes via a respective signal cable of the plurality of signal cables. The computing device may comprise a memory and a processing device operatively coupled to the memory, the processing device to perform, using the four or more electrodes, an electrocardiogram (ECG) of the user.

FIG. 3A

**Description**

**CROSS-REFERENCE**

This application claims the benefit of U.S. Patent Application No. 17/553,414, filed December 16, 2021 and entitled "TWELVE-LEAD ELECTROCARDIOGRAM DEVICE USING ELECTRODES COUPLED BY A SINGLE CABLE," the disclosure of which is hereby incorporated by reference.

**TECHNICAL FIELD**

The present disclosure relates to medical devices, systems, and methods and in particular, to a small form-factor device for providing electrocardiogram (ECG) monitoring.

**BACKGROUND**

Cardiovascular diseases are the leading cause of death in the world. In 2008, 30% of all global death can be attributed to cardiovascular diseases. It is also estimated that by 2030, over 23 million people will die from cardiovascular diseases annually. Cardiovascular diseases are prevalent across populations of first and third world countries alike, and affect people regardless of socioeconomic status.

Arrhythmia is a cardiac condition in which the electrical activity of the heart is irregular or is faster (tachycardia) or slower (bradycardia) than normal. Although many arrhythmias are not life-threatening, some can cause cardiac arrest and even sudden cardiac death. Indeed, cardiac arrhythmias are one of the most common causes of death when travelling to a hospital. Atrial fibrillation (A-fib) is the most common cardiac arrhythmia. In A-fib, electrical conduction through the ventricles of heart is irregular and disorganized. While A-fib may cause no symptoms, it is often associated with palpitations, shortness of breath, fainting, chest pain or congestive heart failure and also increases the risk of stroke. A-fib is usually diagnosed by taking an electrocardiogram (ECG) of a subject. To treat A-fib, a patient may take medications to slow heart rate or modify the rhythm of the heart. Patients may also take anticoagulants to prevent stroke or may even undergo surgical intervention including cardiac ablation to treat A-fib. In another example, an ECG may provide decision support for Acute Coronary Syndromes (ACS) by interpreting various rhythm and morphology conditions, including Myocardial Infarction (MI) and Ischemia.

Often, a patient with A-fib (or other type of arrhythmia) is monitored for extended periods of time to manage the disease. For example, a patient may be provided with a Holter monitor or other ambulatory electrocardiography device to continuously monitor the electrical activity of the cardiovascular system for e.g., at least 24 hours. Such monitoring can be critical in detecting conditions such as acute coronary syndrome (ACS), among others.

The American Heart Association and the European Society of Cardiology recommends that a 12-lead ECG should be acquired as early as possible for patients with possible ACS when symptoms present. Prehospital ECG has been found to significantly reduce time-to-treatment and shows better survival rates. The time-to-first-ECG is so vital that it is a quality and performance metric monitored by several regulatory bodies. According to the national health statistics for 2015, over 7 million people visited the emergency department (ED) in the United States (U.S.) with the primary complaint of chest pain or related symptoms of ACS. In the US, ED visits are increasing at a rate of or 3.2% annually and outside the U.S. ED visits are increasing at 3% to 7%, annually.

**BRIEF DESCRIPTION OF THE DRAWINGS**

The novel features of the disclosure are set forth with particularity in the appended claims. A better understanding of the features and advantages of the present disclosure will be obtained by reference to the following detailed description that sets forth illustrative embodiments, in which the principles of the invention are utilized, and the accompanying drawings of which:

FIG. 1 is a diagram illustrating electrocardiogram (ECG) waveforms, in accordance with some embodiments of the present disclosure.
FIG. 2 illustrates a single dipole heart model with a 12 lead set represented on a hexaxial system, in accordance with some embodiments of the present disclosure.
FIG. 3A is a diagram illustrating an ECG monitoring device, in accordance with some embodiments of the present disclosure.
FIG. 3B is a hardware block diagram of a computing device of the ECG monitoring device of FIG. 3A, in accordance with some embodiments of the present disclosure.
FIG. 3C is a diagram illustrating an internal view of a housing cable of the ECG monitoring device of FIG. 3A, in accordance with some embodiments of the present disclosure.

FIGS. 3D is a diagram illustrating the ECG monitoring device with an internal view of the housing cable, in accordance with some embodiments of the present disclosure

FIG. 4A is a system block diagram illustrating an of an ECG monitoring device, in accordance with some embodiments of the present disclosure.

FIG. 4B is a system block diagram illustrating an of an ECG monitoring device, in accordance with some embodiments of the present disclosure.

FIG. 5A illustrates the ECG monitoring device of FIG. 3A in operation, in accordance with some embodiments of the present disclosure.

FIG. 5B illustrates the ECG monitoring device of FIG. 3A in operation, in accordance with some embodiments of the present disclosure.

FIG. 5C illustrates the ECG monitoring device of FIG. 3A in operation, in accordance with some embodiments of the present disclosure.

FIG. 5D illustrates the ECG monitoring device of FIG. 3A in operation, in accordance with some embodiments of the present disclosure.

FIG. 5E is a diagram illustrating the front view of an ECG monitoring device, in accordance with some embodiments of the present disclosure.

FIG. 5F is a diagram illustrating the front view of an ECG monitoring device, in accordance with some embodiments of the present disclosure.

FIGS. 6A-D illustrates an ECG monitoring device in operation, in accordance with some embodiments of the present disclosure.

FIG. 7A illustrates a comparison of an ECG pattern of converted leads with an ECG pattern of measured leads, in accordance with some embodiments of the present disclosure.

FIG. 7B illustrates a comparison of an ECG pattern of converted leads with an ECG pattern of measured leads with respect to an R-wave, in accordance with some embodiments of the present disclosure.

FIG. 8 is a block diagram of an example computing device that may perform one or more of the operations described herein, in accordance with some embodiments of the present disclosure.

## DETAILED DESCRIPTION

It is to be understood that the present disclosure is not limited in its application to the details of construction, experiments, exemplary data, and/or the arrangement of the components set forth in the following description. The embodiments of the present disclosure are capable of other embodiments or of being practiced or carried out in various ways. Also, it is to be understood that the terminology employed herein is for purpose of description and should not be regarded as limiting.

In the following detailed description of embodiments of the present disclosure, numerous specific details are set forth in order to provide a more thorough understanding of the disclosure. However, it will be apparent to one of ordinary skill in the art that the concepts within the disclosure can be practiced without these specific details. In other instances, well-known features have not been described in detail to avoid unnecessarily complicating the description.

An electrocardiogram (ECG) provides a number of ECG waveforms that represent the electrical activity of a person's heart. An ECG monitoring device may comprise a set of electrodes for recording these ECG waveforms (also referred to herein as "taking an ECG") of the patient's heart. The set of electrodes may be placed on the skin of the patient in multiple locations and the electrical signal (ECG waveform) recorded between each electrode pair in the set of electrodes may be referred to as a lead. Varying numbers of leads can be used to take an ECG, and different numbers and combinations of electrodes can be used to form the various leads. Example numbers of leads used for taking ECGs are 1, 2, 6, and 12 leads.

The ECG waveforms (each one corresponding to a lead of the ECG) recorded by the ECG monitoring device may comprise data corresponding to the electrical activity of the person's heart. A typical heartbeat may include several variations of electrical potential, which may be classified into waves and complexes, including a P wave, a QRS complex, a T wave, and a U wave among others, as is known in the art. Stated differently, each ECG waveform may include a P wave, a QRS complex, a T wave, and a U wave among others, as is known in the art. The shape and duration of these waves may be related to various characteristics of the person's heart such as the size of the person's atrium (e.g., indicating atrial enlargement) and can be a first source of heartbeat characteristics unique to a person. The ECG waveforms may be analyzed (typically after standard filtering and "cleaning" of the signals) for various indicators that are useful in detecting cardiac events or status, such as cardiac arrhythmia detection and characterization. Such indicators may include ECG waveform amplitude and morphology (e.g., QRS complex amplitude and morphology), R wave-ST segment and T wave amplitude analysis, and heart rate variability (HRV), for example.

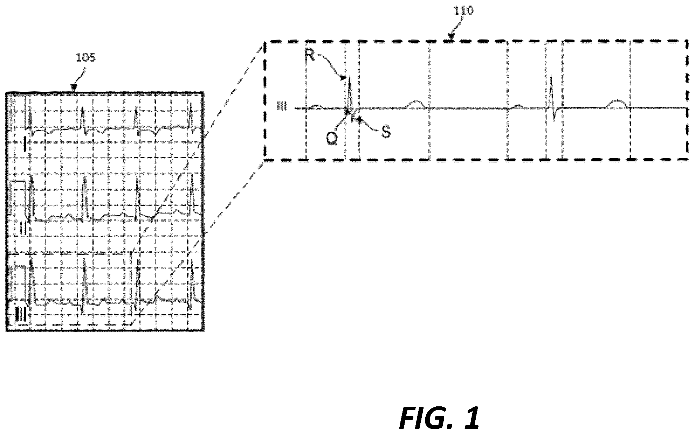

As noted above, ECG waveforms are generated from measuring multiple leads (each lead formed by a different electrode pair), and the ECG waveform obtained from each different electrode pair/lead may be different/unique (e.g., may have different morphologies/amplitudes). This is because although the various leads may analyze the same electrical events, each one may do so from a different angle. FIG. 1A illustrates a view 105 of an ECG waveform detected by each of 3 leads (I, II, and III) when a 3-lead ECG is taken as well as an exploded view 110 of the ECG waveform measured by lead III illustrating the QRS complex. As shown, the amplitudes and morphologies of the ECG waveform taken from leads I - III are all different, with the ECG waveform measured by lead III having the largest amplitude and the ECG waveform measured by lead I having the smallest amplitude.

There are different "standard" configurations for electrode placement that can be used to place electrodes on the patient. For example, an electrode placed on the right arm can be referred to as RA. The electrode placed on the left arm can be referred to as LA. The RA and LA electrodes may be placed at the same location on the left and right arms, preferably near the wrist in some embodiments. The leg electrodes can be referred to as RL for the right leg and LL for the left leg. The RL and LL electrodes may be placed on the same location for the left and right legs, preferably near the ankle in some embodiments. Lead I is typically the voltage between the left arm (LA) and right arm (RA), e.g. $I = LA - RA$. Lead II is typically the voltage between the left leg (LL) and right arm (RA), e.g. $II = LL - RA$. Lead III is the typically voltage between the left leg (LL) and left arm (LA), e.g. $III = LL - LA$. Augmented limb leads can also be determined from RA, RL, LL, and LA. The augmented vector right (aVR) lead is equal to RA - (LA+LL) / 2 or - (I + II) / 2. The augmented vector left (aVL) lead is equal to LA - (RA+LL) / 2 or I - II / 2. The augmented vector foot (aVF) lead is equal to LL - (RA+LA) / 2 or II - I / 2.

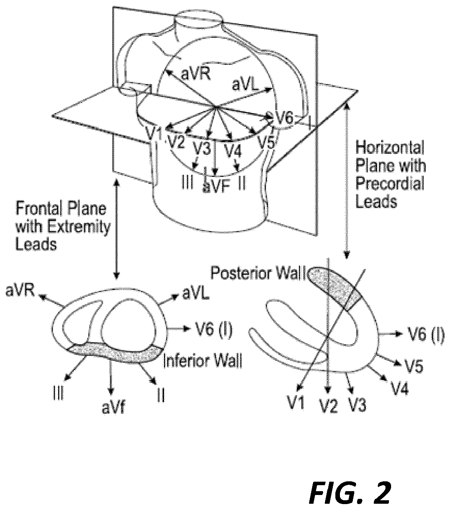

FIG. 2 illustrates a single dipole heart model 115 with a 12 lead set comprising the I, II, III, aVR, aVL, aVF, V1, V2, V3, V4, V5, and V6 leads, all represented on a hexaxial system. The heart model 115 assumes a homogeneous cardiac field in all directions that only changes magnitude and direction with the cycle time. As illustrated in FIG. 2, there are 2 orthogonal planes, the frontal plane and the horizontal plane. Inside each plane, there are several leads to cover the whole plane. In the frontal plane, there are 2 independent leads I and II, and 4 other derived leads III, aVR, aVL, and aVF, each 30 degrees apart. The reason the frontal plane has 2 independent leads is that they are far-field leads, each of which can cover a wider perspective but provide less detail, like a wide-angle camera lens. In the horizontal plane, there are normally 6 independent leads which are all closer to the heart than limb leads and may be referred to as near-field leads. Following the same analogy of a camera, the near-field leads may behave like a zoom-lens that covers less perspective, but with more accuracy towards local activity like ischemia and infarction. The two orthogonal planes are related by using a synthetic reference point formed by Leads I & II, called the Wilson-Central-Terminal (WCT). It is defined as RA + LA + RL/3 but given that both Lead I and II are recorded with reference to the RA so that the voltage of the RA can be considered zero, the WCT (VW) can be calculated using the RA as the reference for both Leads I & II (thus, assuming it to have zero potential) as: Lead I +Lead II/3.

It should be noted that a set of two or more leads may be transformed to generate a full, 12-lead ECG. Such transformation may be performed using a machine learning model (e.g., a neural network, deep-learning techniques, etc.). The machine learning model may be trained using 12-lead ECG data corresponding to a population of individuals. The data, before being input into the machine learning model, may be pre-processed to filter the data in a manner suitable for the application. For example, data may be categorized according to height, gender, weight, nationality, etc. before being used to train one or more machine learning models, such that the resulting one or models are finely-tuned the specific types of individuals. In a further embodiment, the machine learning model may be further trained based on a user's own ECG data, to fine-tune and personalize the model even further to decrease any residual synthesis error.

As discussed herein, a 12-lead ECG should be acquired as early as possible for patients with possible ACS when symptoms present as prehospital ECG has been found to significantly reduce time-to-treatment and shows better survival rates. In addition, current ambulatory ECG devices such as Holter monitors, are typically bulky and difficult for subjects to administer without the aid of a medical professional. For example, the use of Holter monitors requires a patient to wear a bulky device on their chest and precisely place a plurality of electrode leads on precise locations on their chest. These requirements can impede the activities of the subject, including their natural movement such as bathing and showering. Once an ECG is taken by such devices, the ECG is sent to the subject's physician who then analyzes the ECG waveforms and provides a diagnosis and other recommendations. Currently, this process often must be performed through hospital administrators and health management organizations and many patients do not receive feedback in an expedient manner.

A number of handheld ECG measurement devices are known, including devices that may adapt existing mobile telecommunications device (e.g., smartphones) so that they can be used to record ECS. However, such devices either require the use of external (e.g., plug-in) electrodes, or include electrodes in a housing that are difficult to properly hold and apply to the body. Many ECG monitors are also limited to acquiring limb leads (e.g., due to size and other constraints). However, as people age, their QRS and T-wave vector may gradually move from the frontal plane to the horizontal plane, thus increasing the importance of acquiring data from a horizontal plane lead.

As discussed herein, for patients potentially suffering from ACS, including Myocardial Infarction (MI) and Ischemia, a 12 lead ECG should be taken as early as possible to reduce the time to diagnosis and the time to treatment.

The ECG monitoring device in accordance with embodiments of the present disclosure may provide decision support to physicians for ACS from the home of a patient itself, and provides a convenient way for doctors to order 12-lead ECG tests and view reports as often as is necessary for them to manage the health of their patients, especially if they suspect ACS. In addition, an ECG monitoring device in accordance with embodiments of the present disclosure may prevent a patient from undergoing the inconvenience and disruption of an office visit and may save the cost and time of utilizing an ECG technician in the physician's office.

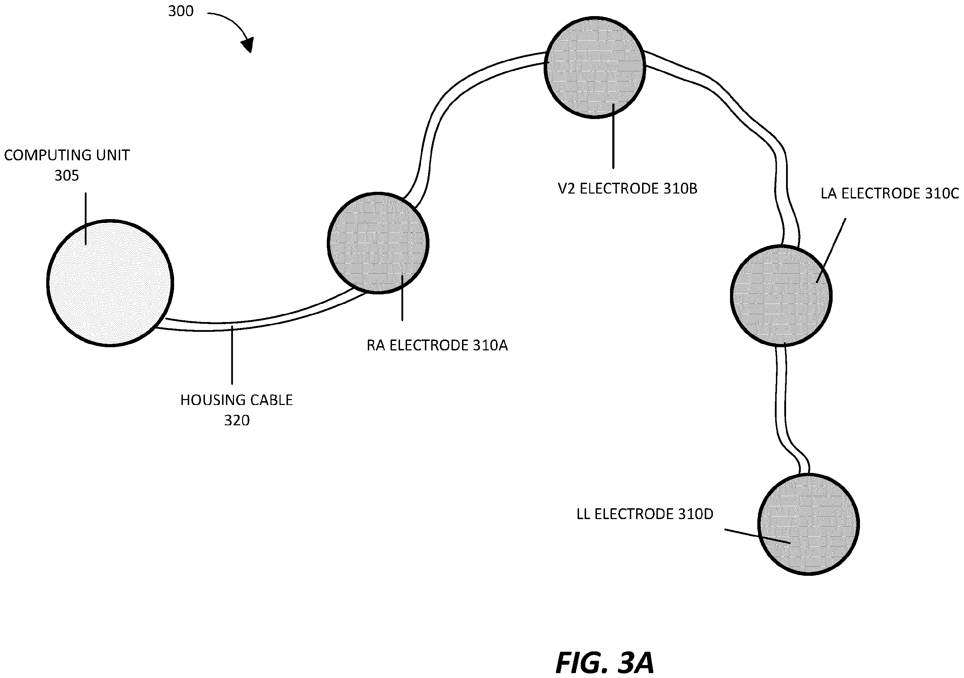

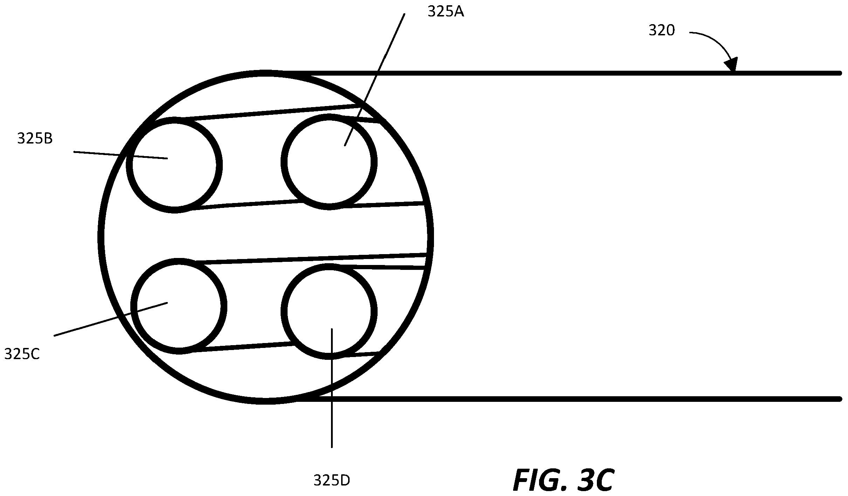

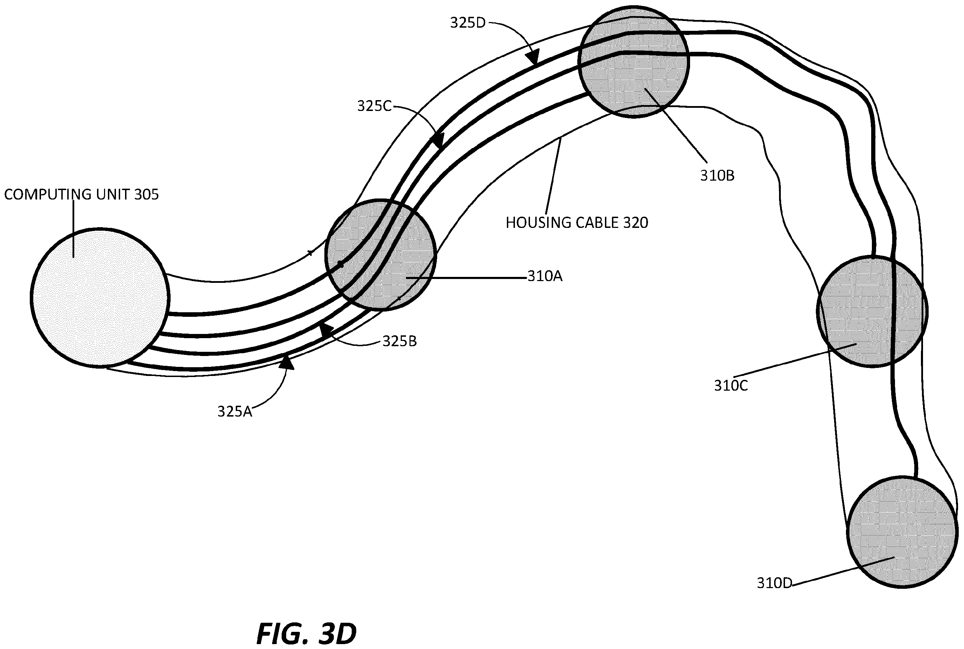

FIG. 3A shows an ECG monitoring device 300 in accordance with some embodiments of the present disclosure. The ECG monitoring device 300 may comprise a computing device 305, a housing cable 320, and a set of electrodes 310A - 310D. Each of the electrodes 310 as well as the computing device 305 may be mounted on and operatively coupled to the housing cable 320. In addition, the housing cable 320 may comprise a set of signal cables 325 (illustrated in FIGS. 3C and 3D), each of which may couple a respective electrode 310 to the computing device 305 as discussed in further detail with respect to FIGS 3C and 3D. In the example of FIG. 3A, the electrode 310A may be a right arm (RA) electrode, the electrode 310B may be a V2 electrode, the electrode 310C may be a left arm (LA) electrode, and the electrode 310D may be a left leg (LL) electrode. The electrode 310A and the electrode 310C may each be positioned anywhere on a respective arm of the user from the fingers to the upper chest region by the shoulders. The position of the LL electrode can be anywhere from the toes to the lower left quadrant of the torso near the groin. As shown in FIG. 3A, the computing device 305 and electrodes 310A-310D are implemented in a circular shape, however the computing device 305 and electrodes 310A-310D may be implemented or realized in any appropriate shape and using any appropriate material. Each of the electrodes 310 may be a dry or wet electrode. The dry electrodes may be coupled to or integrated with clamps, suction cups, bands (e.g., wrist bands, leg bands), cuff braces, or any other appropriate coupling mechanism for coupling to a user.

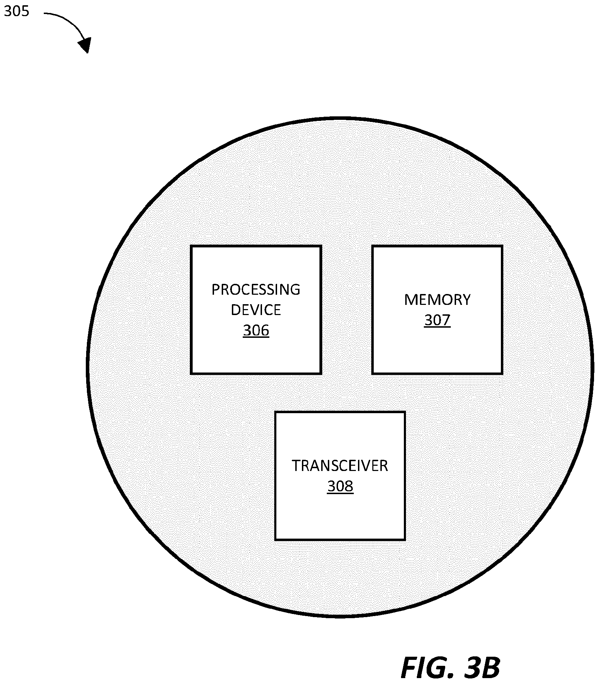

The computing device 305 may comprise hardware to perform the functions described herein. FIG. 3B illustrates a hardware block diagram of computing device 305 which may include hardware such as processing device 306 (e.g., processors, central processing units (CPUs)), memory 307 (e.g., random access memory (RAM), storage devices (e.g., hard-disk drive (HDD)), solid-state drives (SSD), etc.), and other hardware devices (e.g., analog to digital converter (ADC) etc.). A storage device may comprise a persistent storage that is capable of storing data. A persistent storage may be a local storage unit or a remote storage unit. Persistent storage may be a magnetic storage unit, optical storage unit, solid state storage unit, electronic storage units (main memory), or similar storage unit. Persistent storage may also be a monolithic/single device or a distributed set of devices. In some embodiments, the processing device 306 may utilize the electrodes 310 to perform an ECG of a user to which the ECG monitoring device 300 is coupled and then perform basic signal processing of the analog signals measured by each of the electrodes 310. The computing device 305 may include an ADC (not shown) having a high enough sampling frequency for accurately converting the analog signals measured by the set of electrodes into digital signals (e.g., a 24bit ADC operating at 500Hz or higher) for processing by the processing device 306. In some embodiments, the ADC may be a component of the processing device 306. The computing device 305 may generate ECG data by calculating the potential difference for a set of leads. For example, the processing device 306 may calculate LA - RA, RA - V2, RA + LA - V, as well as determine WCT and calculate WCT - V2 and WCT - V4. In some embodiments, the processing device 306 may comprise a dedicated ECG waveform processing and analysis chip that provides built-in leads off detection.

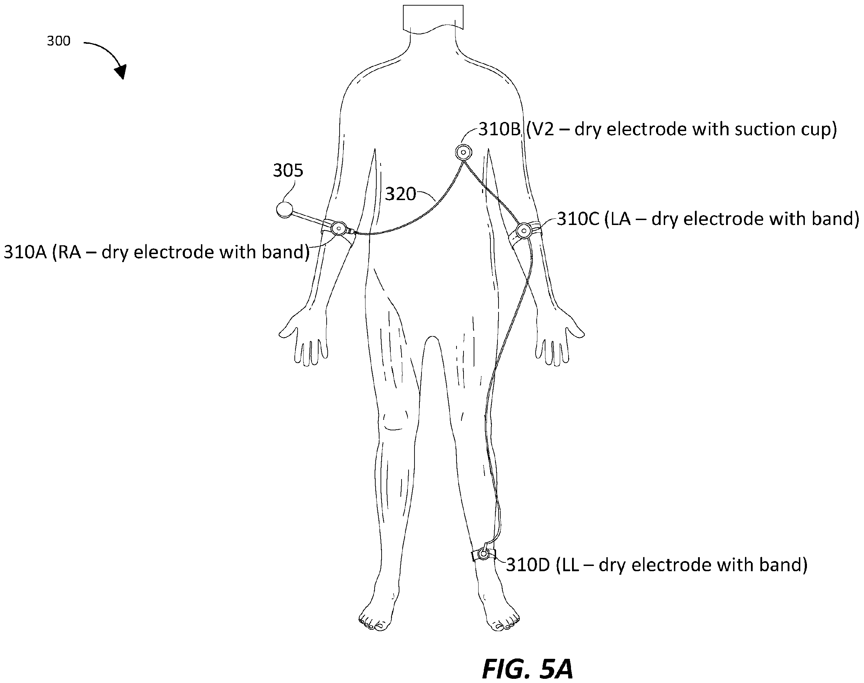

The positioning of the electrodes 310 allows the user to connect the ECG monitoring device 300 to themselves such that each electrode 310 contacts the appropriate location (e.g., as indicated in and discussed in further detail with respect to FIG. 5A). Once connected to the user, the positioning of the electrodes along the housing cable 320 may allow the user to lay in a relaxed supine position while the ECG monitoring device 300 performs an ECG. This is important because muscular activity (e.g., any kind of motion or use of any muscle) may generate an action potential that is larger than a potential produced by the heart. Thus, if the user is moving around (e.g., exerting force on certain electrodes to maintain contact with parts of their body), the muscular activity of such movement may generate potentials that will drown out the potentials from the heart that is are subject of an ECG. Allowing the user to lay in a relaxed position eliminates any stray potentials (e.g., muscle noise (EMG) or motion artifacts) that could interfere with the signals measured by each electrode 310.

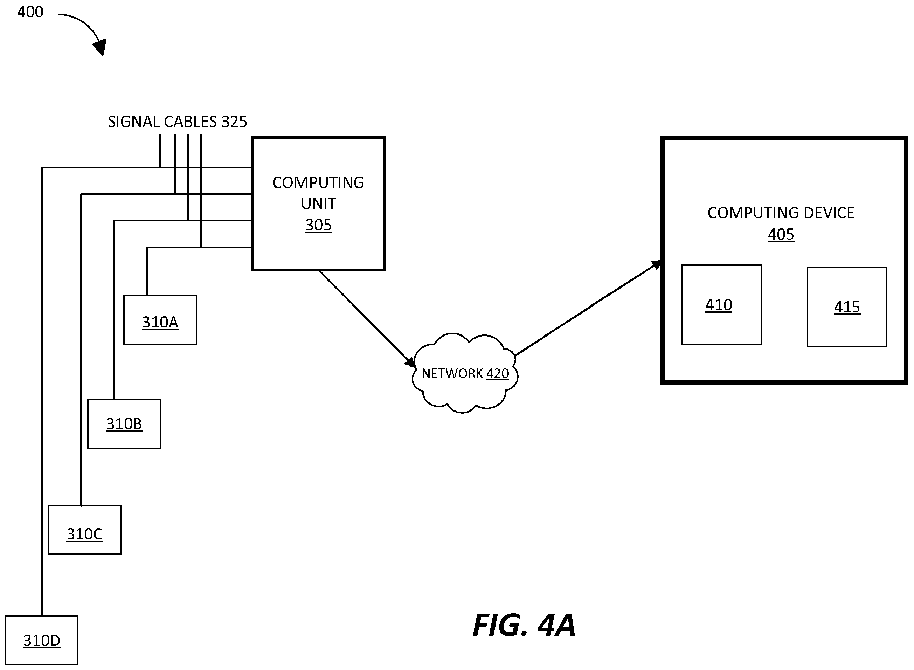

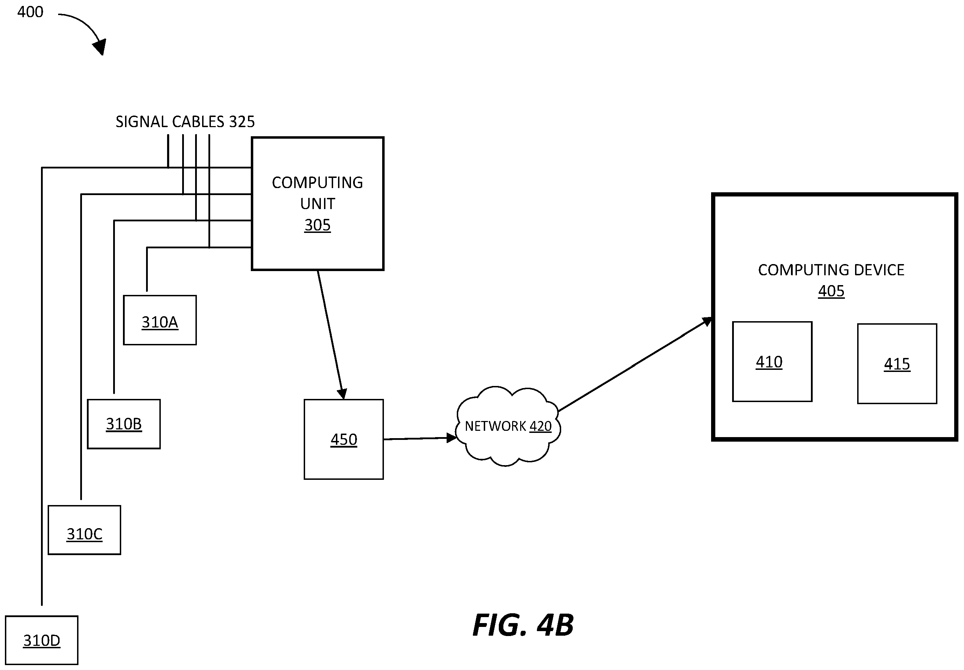

The computing device 305 may further comprise a transceiver 308, which in some embodiments may implement any appropriate protocol for transmitting the ECG data to a computing device 405 (which may represent e.g., a cloud service) or a mobile computing device 450 (which may be remote from the ECG monitoring device 300 as illustrated in FIGS. 4A and 4B) either directly or via a network. For example, the transceiver 308 may include (or be coupled to) a network interface device (not shown) configured to connect with a network 420 (illustrated in FIGS. 4A and 4B) in order to transmit the signal processed data to a computing device 405 either directly or via a mobile computing device 450. The network 420 may be a cellular data network (e.g., using GSM, GSM plus EDGE, CDMA, quadband, or other cellular protocols) or a WiFi (e.g., an 802.11 protocol) network. In some embodiments, the transceiver 308 may transmit the ECG data to the mobile computing device 450 which may in turn transmit the ECG data to the computing device 405 as shown in FIG. 4B. For example, the transceiver 308 may comprise a Bluetooth™ chip for transmitting ECG data via Bluetooth to mobile computing device 450 (e.g., a laptop or smart phone of the user).

FIG. 3C depicts an internal view of the housing cable 320, and shows the set of signal cables 325A - 325D that couple the electrodes 310 to the computing device 305. Each of the signal cables 325 may be any appropriate cable which can facilitate the transfer of data between the electrodes 310 and the computing device 305 using any appropriate data transfer protocol such as e.g., universal serial bus (USB). For example, each of the set of signal cables 325 may comprise a USB cable and each of the electrodes 310 may comprise a USB socket (not shown), while the computing device 305 may comprise multiple USB sockets (not shown) for each of the set of signal cables 325. It should be noted that in some embodiments, the housing cable 320 may not be removable and may be permanently integrated to the electrodes 310 and the computing device 305. In other embodiments, the housing cable may be removably coupled to the electrodes 310 and the computing device 305.

FIG. 3D illustrates the ECG monitoring device 300 with an internal view of the housing cable 320 and the internal connections between each electrode 310 and the computing device 305 via a respective signal cable 325 in the housing cable 320. As can be seen in FIG. 3D, signal cable 325A connects electrode 310A to the computing device 305, signal cable 325B connects electrode 310B to the computing device 305, signal cable 325C connects electrode 310C to the computing device 305, and signal cable 325D connects electrode 310D to the computing device 305. It should be noted that signal cables 325 illustrated as passing through a particular electrode 310 do not actually pass through that electrode, as each electrode 310 is integrated to an exterior of the housing cable 320 and connected to the computing device 305 via a particular signal cable 325 that runs through the housing cable 325. Additionally, in other embodiments, there may be one or more signal cables 325 to couple the electrodes 310 to one another.

FIG. 4A illustrates a system block diagram of the ECG monitoring device 300 without the housing cable 320. Each of the electrodes 310 may be coupled individually to the computing device 305 via a respective signal cable 325. As discussed herein, the processing device 306 within the computing device 305 may perform basic signal processing of the analog signals received from the electrodes 310 and generate ECG data (by calculating the potential difference for each lead being measured) including a waveform for each lead being measured. As discussed herein, in some embodiments the processing device 306 may then transmit the ECG data via transceiver 308, to a computing device 405 (which may represent e.g., a cloud service). The computing device 405 may include a lead synthesis software module 410 and an ECG waveform interpretation software module 415. The computing device 405 may execute the lead synthesis software 410 to synthesize (based on the received ECG data) ECG waveforms corresponding to leads that were not measured by the electrodes 310 of the ECG monitoring device 300 as discussed in further detail herein. The processing device 305 may execute the ECG waveform interpretation software 415 to generate diagnostic interpretations based on the measured and synthesized ECG waveforms, as discussed in further detail herein. After the computing device 405 has executed the lead synthesis software 410 and the ECG waveform interpretation software 415, it may transmit the results to a computing device (e.g., computing device of a physician or healthcare provider, or a mobile device/laptop of the user).

FIGS. 5A-5D illustrate the ECG monitoring device 300 in operation. As can be seen, each of the electrodes 310 as well as the computing device 305 may be coupled to the user via any appropriate coupling mechanism. To take an ECG, the user may connect the ECG monitoring device 300 to themselves using the coupling mechanisms as shown to apply each electrode 310 (as well as the computing device 305) to the appropriate location as indicated in FIGS. 5A - 5D. The ECG monitoring device 300 may connect to the user in such a way as to allow the user to lie down in a relaxed position while an ECG is being taken. This is important because muscular activity (e.g., any kind of motion or use of any muscle) may generate an action potential that is larger than a potential produced by the heart. Thus, if the user is moving around (e.g., exerting force on certain electrodes to maintain contact with parts of their body) they will generate potentials that will drown out the potentials from the heart that is the subject of an ECG. Allowing the user to lay in a relaxed position eliminates any stray potentials (e.g., muscle noise (EMG) or motion artifacts) that could interfere with the signals measured by each electrode 310 and allows the user to be in a relaxed position. The user does not have to hold or exert force on any component of the ECG monitoring device 300 (or do anything that would require muscular activity) once it is connected to them.

Removing the EMG and motion artifacts allows for a cleaner signal and greater diagnostic quality. The user may position the electrodes such that electrodes are in contact with the RA, V2, LA, and LL positions respectively. In some examples, a mobile computing device 405 alerts the user to the appropriate location to place the electrodes on the body of the user. In another example, the chest and arm locations are marked and shown by a healthcare professional who prescribes the ECG monitoring device 300 to the patient. In some embodiments, one or more of the electrodes 310 of the ECG monitoring device 300 may comprise dry electrodes, which are easier for at home use because they stay in place more easily and have a longer lifetime use. Dry electrodes tend to give lower signal quality due to a lower level of conductive contact. In other embodiments, one or more of the electrodes 310 may be wet electrodes, e.g., when the ECG monitoring device 300 is being operated by a healthcare professional or the user has been trained to use wet electrodes. The wet electrodes may provide better conductive contact and produce a higher quality signal. The length of the housing cable 320 may accommodate users of various sizes. The housing cable 320 may have a length that corresponds to (e.g., fits with no slack between electrodes 310) a user with a particular height (e.g., six foot 2 inches), users that are smaller in height than the height the housing cable 320 corresponds to may simply have additional slack between electrodes once the ECG monitoring device 300 has been coupled to them.

In some embodiments, the housing cable 320 may have elastic properties and may have a length corresponding to a user with a smaller particular height (e.g., 4 foot 7 inches). The signal cables 325 may each be implemented using a flexible printed circuit (not shown) with an over mold (not shown) comprising e.g., a thermoplastic polyurethane material or any other appropriate material to provide a conductive channel through which analog signals generated by an electrode 310 may be routed to the computing device 305. In some embodiments, all of the electrodes 310 may be implemented using a single flexible printed circuit that runs the length of the housing cable 320. In these embodiments, the closer the individual is to the height the housing cable 320 corresponds to, the less tension will be applied the housing cable 320.

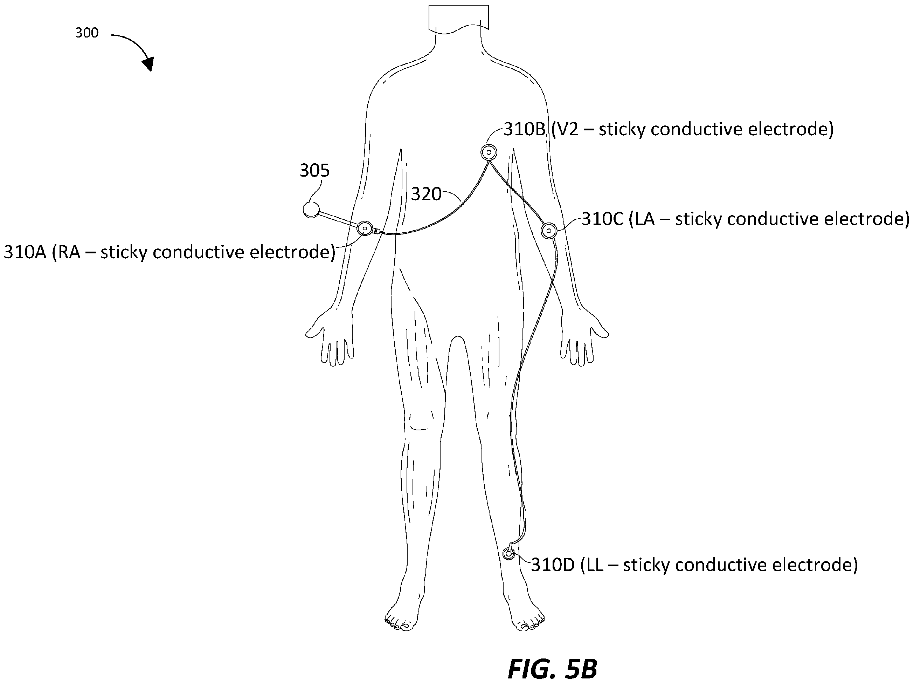

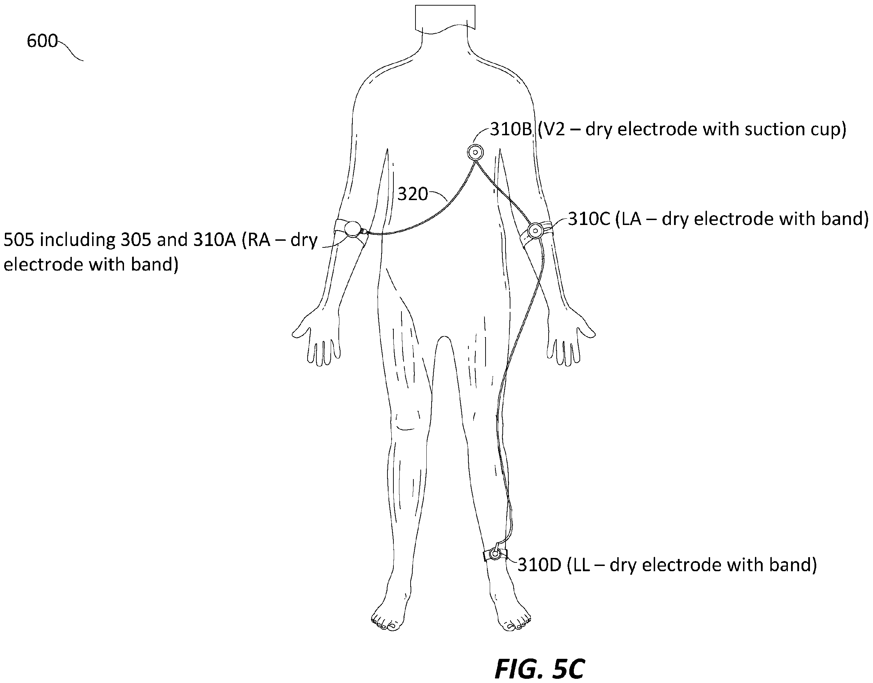

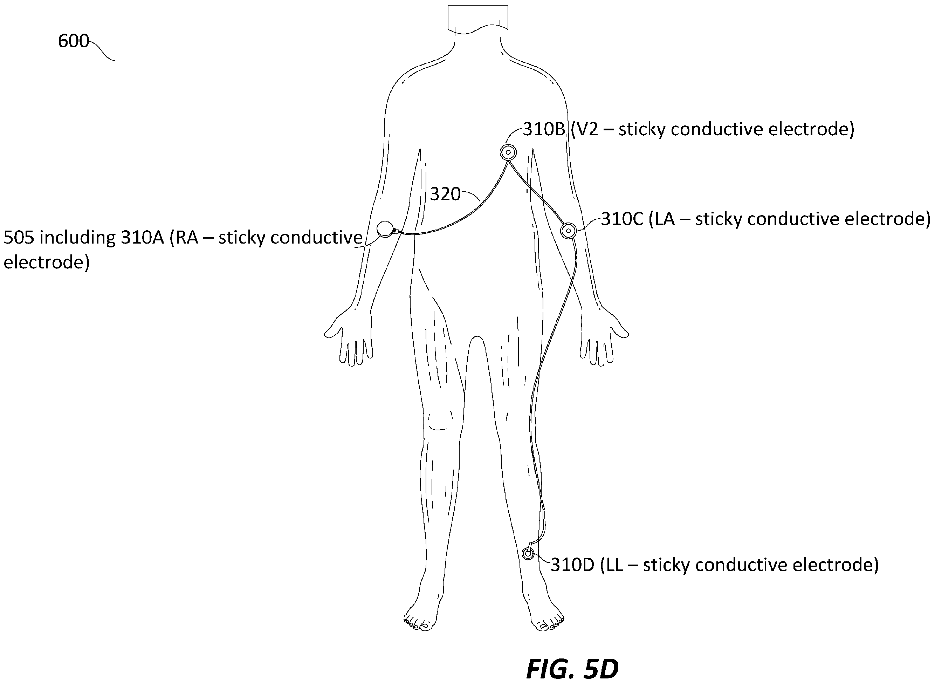

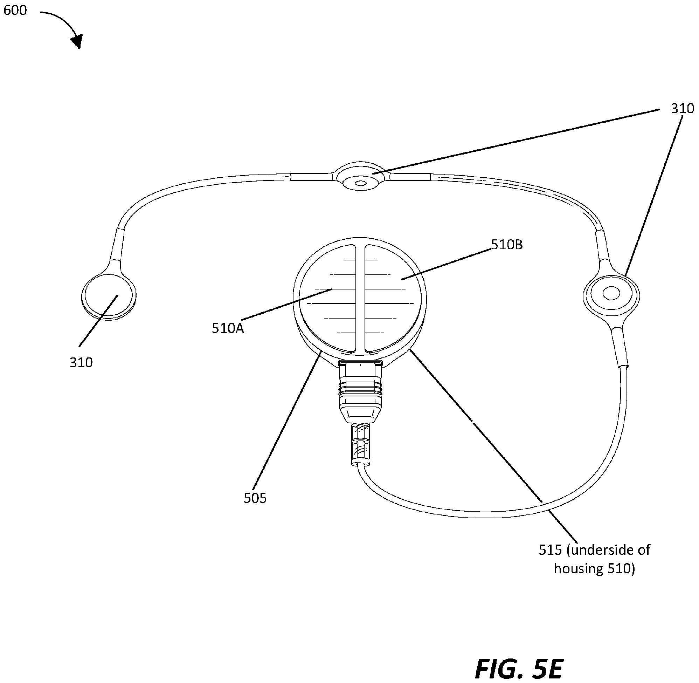

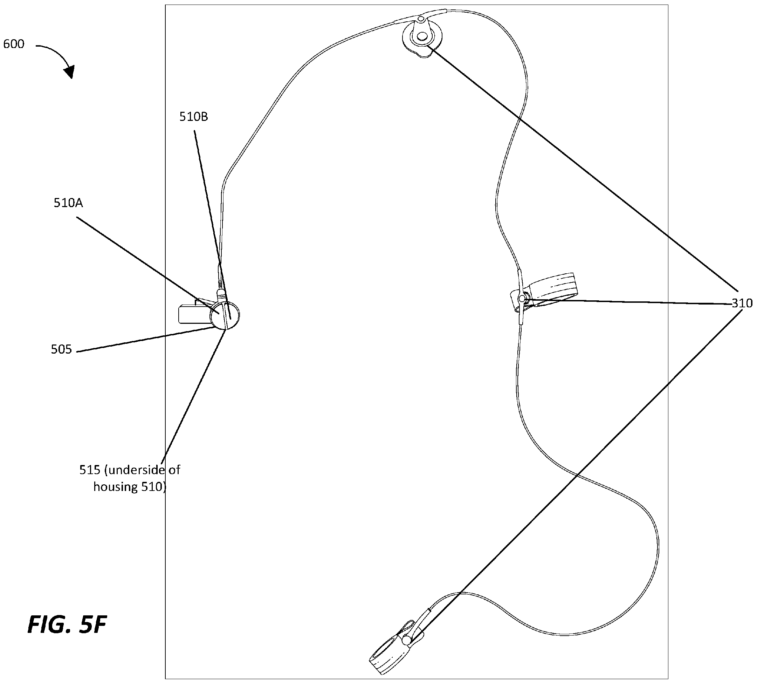

In the example FIG. 5A the electrodes 310A, 310C, and 310D may be dry electrodes connected to the user by a band, while the electrode 310B (the chest electrode) is a dry electrode that is coupled to the user via a suction cup. The computing device 305 may also couple to the user via a band. In the example of FIG. 5B, the electrodes 310 may each comprise a wet sticky conductive electrode that couples to the user via the adhesive properties of the electrodes, while the computing device 305 couples to the user via a band (or other appropriate coupling mechanism). It should be noted that any combination of wet and dry electrodes can be used regardless of the configuration. FIGS. 5C and 5D illustrate embodiments of the present disclosure that utilize similar configuration as FIGS. 5A and 5B respectively, except that the computing device 305 and the electrode 310 are integrated together into a single unit 505 as shown in FIGS. 5E and 5F.

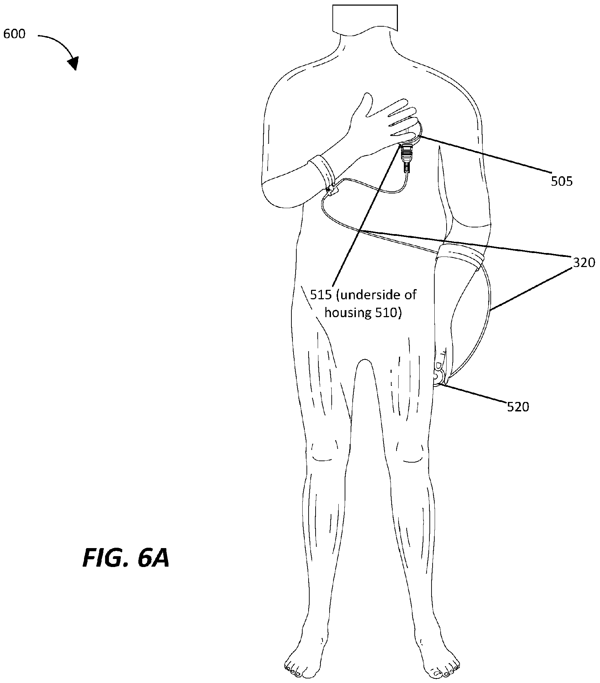

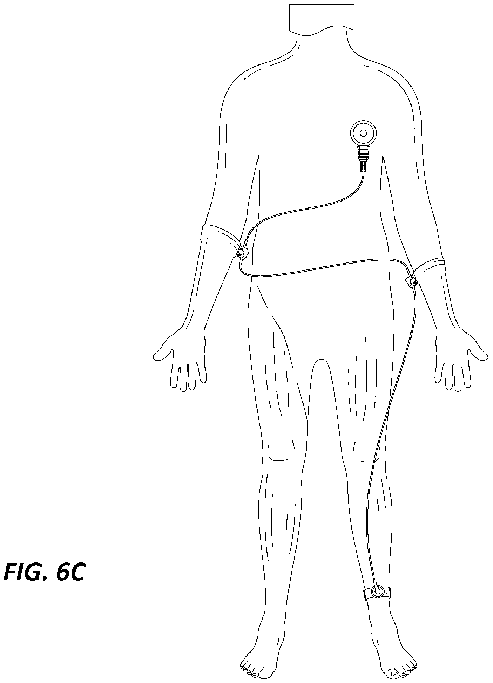

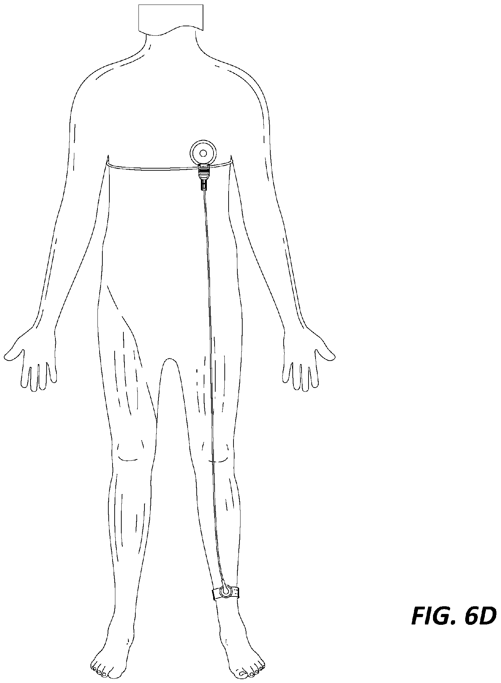

FIGS. 5E and 5F illustrate an ECG monitoring device 600 which may correspond to the ECG monitoring device shown in FIGS. 5C and 5D and which may comprise a housing cable 320, computing device 505, and one or more electrodes 310. The computing device 505 may provide the functionality of the computing device 305 and the electrode 310A together in a single unit. The computing device 505 may comprise a single housing 510 upon which electrodes 510A and 510B may be mounted. The electrodes 510A and 510B may be mounted on a top surface of the housing 510. The computing device 505 may further comprise an electrode 515 which may be mounted on a bottom surface of the housing 510. The housing 510 may include hardware as described herein with respect to FIG. 3B. In some embodiments, the electrodes 510A and 510B may correspond to RA electrodes and the electrode 515 may be a V2 electrode. In some embodiments, an ECG monitoring device 600 may comprise a housing cable 320, computing device 505, and another dual-sided electrode housing 520 that may be similar to the computing device 505 but without the hardware described with respect to FIG. 3B. The user may contact the ECG monitoring device 600 as shown in FIG. 6A.

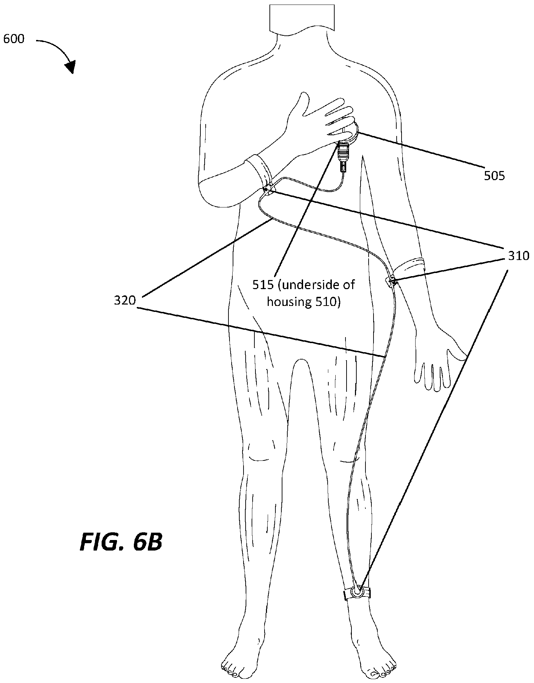

FIG. 6A-6D illustrate further examples of the ECG monitoring device 600 in operation. For example, FIG. 6A illustrates an embodiment where the ECG monitoring device 600 is comprised of computing device 505, and another dual-sided electrode housing 520 that may be similar to the computing device 505 but without the hardware described with respect to FIG. 3B. Both the computing device 505 and dual-sided electrode housing 520 may comprise one or more electrodes on the top and bottom of the housing. The electrode 515 (not shown) positioned on the underside of the housing 510 may make contact with the V2 position and the electrodes 510A and 510B positioned on the top of the housing 510 may make contact with the right hand or fingers (RA position) of the user. The housing 520 may be positioned such that an electrode (not shown) on the underside of the housing 520 makes contact with the left groin (LL position) and an electrode (not shown) on the top side of the housing 520 makes contact with their left hand or fingers (LA position). Each of the housings 510 and 520 are connected by the housing cable 320 and the signal cables 325 within the housing cable 320. It should be noted that these embodiments require muscular activity by the user to maintain contact between the computing device 505, housing 510, and their body, unlike embodiments of the present disclosure described with respect to FIGS. 3 and 5A - 5D. This embodiment thereby enables the ECG monitoring device 600 to take a four channel ECG. In the example of FIG. 6B, the ECG monitoring device 600 comprises the computing device 505 that is positioned by the user as shown as well as LA and LL electrodes coupled to the user via bands to take a four channel ECG.

Referring back to FIG. 4, and as discussed herein, the computing device 405 may execute the lead synthesis software 410 in order to synthesize a full 12 lead set from the set of leads measured by the ECG monitoring device 300. The lead synthesis software 410 may comprise a lead conversion ML model which may function to synthesize the V1, V3, V4, V5, and V6 leads based on one or more of the measured/derived I, II, III, aVR, aVL, aVF, and V2 leads. In some embodiments, the lead conversion ML model may comprise a single dipole global model, that provides a "one size fits all" approach to lead conversion. A conversion model can be expressed mathematically as:

$$Vpred = f(W, Vx)$$

Where Vpred are the predicted V leads, f() is a transfer function with input leads Vx (I, II, and V2 in this case), and coefficients (W). Appropriate coefficients W that will minimize the error between Vpred and actual sampled V lead signals (Vreal) (Min err = E|(Vreal - Vpred)| ^2) must be found.

Locating such appropriate coefficients W is a problem that may be solved using any appropriate method such as a supervised learning task or a curve fitting problem. In some embodiments, the lead synthesis software 410may utilize linear optimization and the least square method (LS):

$$Vpred = f(W, Vx) \; -\!-> Vpred = Vx \; W$$

By stacking many paired samples of Vx to form a matrix X and Y (given as Y = X.W), linear optimization and the LS method can be used to solve for W. More specifically, processing device 206 may prepare the matrix X and Y for Y = X.W, using the LS method to obtain a conversion coefficient:

$$X' = X^\wedge t$$

The covariance of X may then be given as:

$$CovX = X'X$$

W may then be calculated as follows:

$$Xinv = inv(CovX)$$

$$W = CovX\_inv * X' * Y,$$

(here we assume CovX is a full rank matrix).

Training data comprising e.g., 100,000 ECGs with average beats may be used for the training of the lead conversion ML model. The final conversion model is a matrix W having a 3 by 5 shape. The leads V1, V3, V4, V5, and V6 are predicted using the input leads I, II, V2.

In some embodiments, the computing device 405 may quantify the quality of the lead conversion ML model and determine whether a different lead conversion ML model (e.g., a more individualized model, or a multiple-dipole model) should be used. Techniques for quantifying the quality of a lead conversion ML model may be complicated since most statistical similarity methods are more closely related to amplitudes of every point, like the popular R-square method. However, the overall ECG morphology patterns which are used for interpretations are not based on amplitudes alone. For example, a Q wave is important for myocardial infarction detection, but its amplitude is generally much smaller than an R wave. One way to measure the quality of conversion is to compare the important ECG features used for ECG interpretations. Any appropriate algorithm (such as GE's EK12/12SL algorithm) can be used for this purpose. The data shown in table 1 below was obtained using the R-square ($R^2$) algorithm (shown directly below), however, any appropriate 12-lead measurement algorithm may be used.

$$R^2 = \left\{ 1 - \frac{\sum [Derived(sample\ k) - Measured(sample\ k)]^2}{\sum [Measured(sample\ k)]^2} \right\}$$

Table 1

|         | V1 | V3 | V4 | V5 | V6 |
|---------|----|----|----|----|----|
| $R^2$ (%) | 87 | 82 | 70 | 75 | 78 |

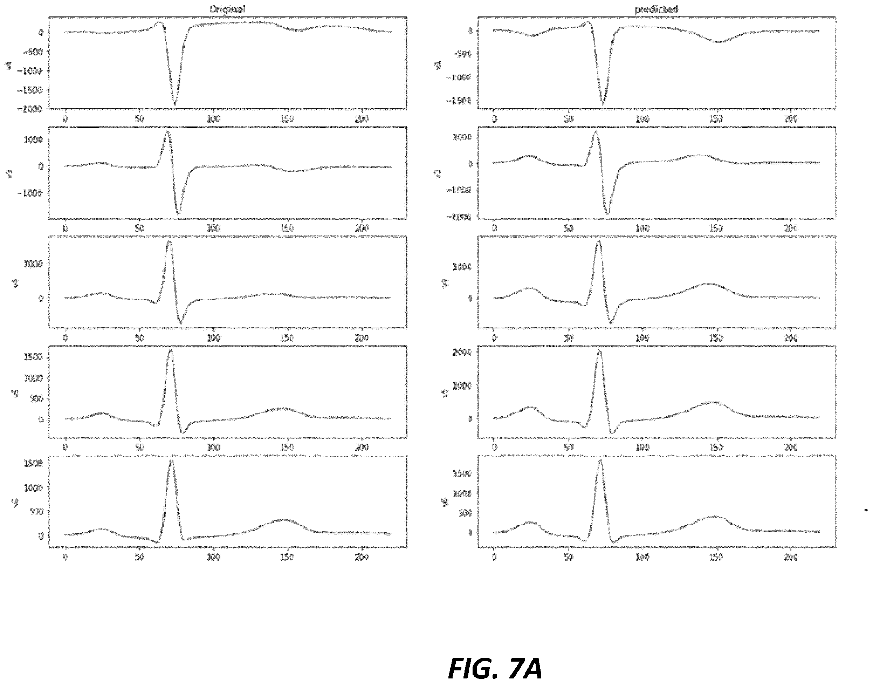

As can be seen, R-V1 is higher than the other V leads. Theoretically, V1 is the most difficult to predict from leads I, II, V2, since it represents more right ventricular activity, while the input leads are more reflective of the left ventricular electric field. The V3 and V4 signals usually have higher amplitudes than other leads due to their proximity to the heart. FIG. 7A illustrates the results of lead conversion when the lead conversion ML model is a single dipole global model, with examples of the measured/original leads (left column) vs. converted/predicted leads (right column). As can be seen in FIG. 7A, the ECG pattern of converted leads accurately follows the ECG pattern for the measured/original leads.

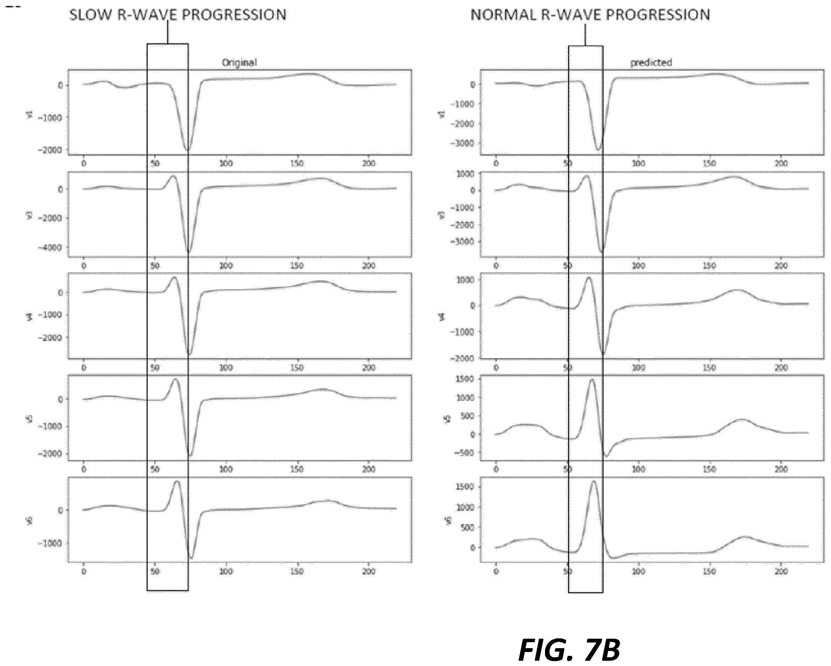

However, the computing device 405 may also determine that the performance provided by the single dipole global model is not sufficient. For example, FIG. 7B illustrates a pattern called 'slow R wave progression' in the measured/original leads (left column), which is a criterion for possible previous anterior infarction, while the converted/predicted leads (right column) have a 'normal' R wave progression from V3-V6. It may be difficult for an ML model that is trained with and follows a global trend of R wave progression of the majority of ECG samples, to avoid such issues. However, in such situations, the computing device 405 may alleviate this problem by selecting a different lead conversion ML model with a higher level of individualization or multiple dipoles.

A single pole cardiac source model may cover all phases of cardiac signal progression, including both atrial and ventricular depolarization and repolarization, while being relatively simple. However, such a model may be oversimplified in certain circumstances since a multiple-dipole model generally provides better accuracy than a single dipole model. Thus, in some embodiments, the computing device 405 may utilize a lead conversion ML model based on a multiple-dipole conversion model. The below table illustrates R-square statistics when a multiple-dipole model is used. As can be seen, both the QRS and ST-T segments show improved accuracy (relative to the single-dipole model in table 1 above), while the P-wave results are not improved. As a result, the lead conversion ML model used by computing device 405 may consider depolarization and repolarization separately.

Table 2

|  | V1 | V3 | V4 | V5 | V6 |
|---|---|---|---|---|---|
| R^2 P (%) | 75 | 80 | 74 | 76 | 73 |
| R^2 QRS (%) | 88 | 84 | 74 | 78 | 78 |
| R^2 St-T (%) | 84 | 89 | 79 | 82 | 81 |

Referring to the basic optimization equation (Vpred = f(W, Vx)), it is clear that both a linear function f() and a non-linear function f() can be used for finding the W. In some embodiments, the computing device 405 may utilize a nonlinear lead conversion model in situations where the increased computational burden required for the use of a nonlinear model is justified by significantly superior performance.

A number of deep learning methods may also be used to synthesize a full 12 lead set from the set of leads measured by the ECG monitoring device 300. For example, the lead conversion ML model may utilize artificial neural networks (ANNs) for supervised classification, where the outcome of the model represents the probability of the input sample to be in a specific class of data or exhibits some peculiar characteristics. In another example, a data driven approach based on convolutional neural networks (CNNs) is used. By using convolution operations, the lead conversion ML model may take into account the correlation among temporally closed input samples to infer a single output data point. More specifically, a single output sample (each precordial lead) at a generic time t is affected by all the input samples (all limb leads) from $t - \tau$ to $t + \tau$. The value of $\tau$, which represents the receptive field of the network, highly depends on the model architecture and typically increases with its depth, i.e., the number of consecutive layers. The ability to generalize on unseen data, and avoid overfitting issues, is of primary importance for all data driven approaches. Complex models, along with small datasets, may lead to excellent performance on the training set, but may perform poorly on unseen data. Any appropriate regularization method may be used to optimize the model, such as inter and intra-layer normalization (e.g., batch normalization and layer normalization), and data augmentation techniques. Finally, to improve the effectiveness and efficiency of the model, the use of residual connections, i.e., an identity mapping that allow gradients to flow through a layer during the backpropagation of gradient-based optimization algorithms may be utilized.

The computing device 405 may execute an ECG waveform interpretation software 415 in order to perform interpretation based on the synthesized full 12-lead set of ECG waveforms using an interpretation ML model. The ECG waveform interpretation software 415 may comprise an interpretation ML model which may function to determine (based on the full 12 lead set measured/generated by the computing device 405) interpretations indicating myocardial ischemia (anterior, lateral, ischemia), myocardial infarction (anterior, lateral mi), left and right bundle branch block and right ventricular hypertrophy, among others. The interpretation ML model may be trained to perform well on morphology-based abnormalities using the converted 12-leads. More specifically, the interpretation ML model may comprise a deep neural network (DNN) model that is trained with converted lead signals, so that it can identify new ECG feature patterns, even if they are not identical with the original ones, thus enabling the interpretation ML model to differentiate among different abnormalities. The interpretation ML model may be a convolutional DNN with 6 residual blocks and 3 fully connected layers. The ML interpretation model may also have dropout and batch normalization layers to improve the generalization.

The interpretation ML model may be trained using a 12-lead ECG database (not shown), which has ECG data for a large number of 12-lead ECGs, each with e.g., 10 seconds of data. The 12-lead ECG database may include ECG data with various types of ECG abnormalities. In the training data, morphology-based ECGs may be clustered into 6 categories: ischemia, infarction, left bundle branch block (LBBB), right bundle branch block (RBBB), left ventricle hypertrophy (LVH), and others. Of those ECGs, a majority may be used for training, while the remainder are used for testing. Experimental data has shown that training the interpretation ML model on a converted lead set optimizes the interpretation performance. Thus, in some embodiments the interpretation ML model may be further trained on a converted lead set to optimize its interpretation performance. More specifically, the interpretation ML model is first trained and tested with the originally sampled 12-lead data and the interpretation performance is recorded. The interpretation ML model is then reinitialized and retrained/retested with converted 12-lead data, and the interpretation performance is recorded.

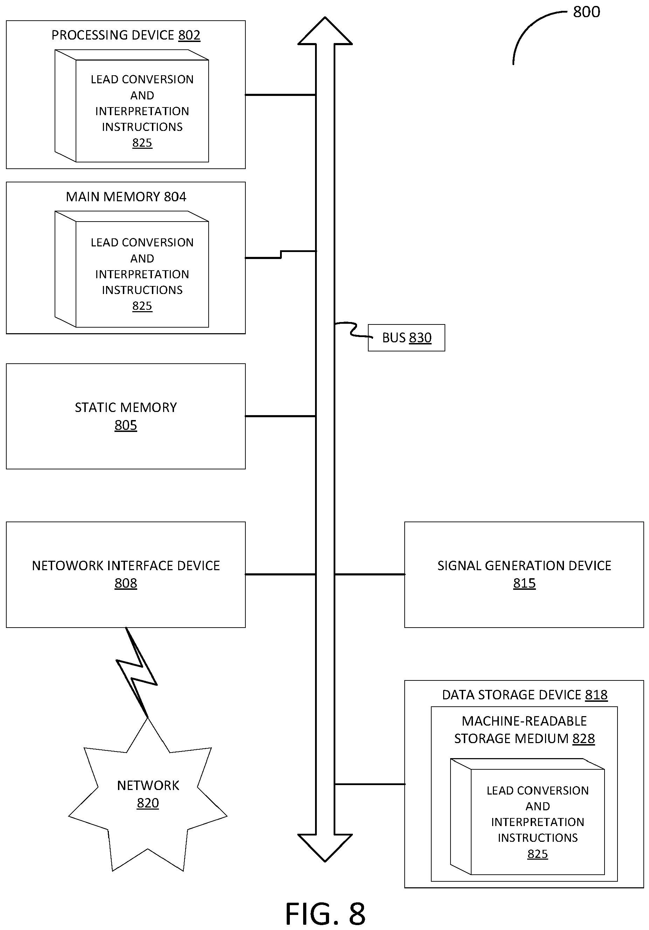

FIG. 8 illustrates a diagrammatic representation of a machine in the example form of a computer system 800 within which a set of instructions, for causing the machine to perform any one or more of the methodologies discussed herein. In alternative embodiments, the machine may be connected (e.g., networked) to other machines in a local area network (LAN), an intranet, an extranet, or the Internet. Further, while only a single machine is illustrated, the term "machine" shall also be taken to include any collection of machines that individually or jointly execute a set (or multiple sets) of instructions to perform any one or more of the methodologies discussed herein.

The exemplary computer system 800 includes a processing device 802, a main memory 804 (e.g., read-only memory (ROM), flash memory, dynamic random access memory (DRAM), a static memory 806 (e.g., flash memory, static random access memory (SRAM), etc.), and a data storage device 818, which communicate with each other via a bus 830. Any of the signals provided over various buses described herein may be time multiplexed with other signals and provided over one or more common buses. Additionally, the interconnection between circuit components or blocks may be shown as buses or as single signal lines. Each of the buses may alternatively be one or more single signal lines and each of the single signal lines may alternatively be buses.

Computing device 800 may further include a network interface device 908 which may communicate with a network 820. Processing device 802 represents one or more general-purpose processing devices such as a microprocessor, central processing unit, or the like. More particularly, the processing device may be complex instruction set computing (CISC) microprocessor, reduced instruction set computer (RISC) microprocessor, very long instruction word (VLIW) microprocessor, or processor implementing other instruction sets, or processors implementing a combination of instruction sets. Processing device 802 may also be one or more special-purpose processing devices such as an application specific integrated circuit (ASIC), a field programmable gate array (FPGA), a digital signal processor (DSP), network processor, or the like. The processing device 802 is configured to execute lead synthesis and interpretation generation instructions 825, for performing the operations and steps discussed herein.

The data storage device 815 may include a machine-readable storage medium 828, on which is stored one or more sets of lead synthesis and interpretation generation instructions 825 (e.g., software) embodying any one or more of the methodologies of functions described herein. The lead synthesis and interpretation generation instructions 825 may also reside, completely or at least partially, within the main memory 804 or within the processing device 802 during execution thereof by the computer system 800; the main memory 804 and the processing device 802 also constituting machine-readable storage media. The lead synthesis and interpretation generation instructions 825 may further be transmitted or received over a network 820 via the network interface device 808.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements, these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical range recited herein is intended to include all sub-ranges subsumed therein.

While preferred embodiments of the present disclosure have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

## Claims

1. An apparatus comprising:

   a housing cable comprising a plurality of signal cables positioned therein;
   four or more electrodes positioned along the housing cable to contact a particular location of a user without any muscular activity of the user when the four or more electrodes are connected to the user; and
   a computing device positioned along the housing cable and operatively coupled to each of the four or more electrodes via a respective signal cable of the plurality of signal cables, the computing device comprising:

      a memory; and
      a processing device operatively coupled to the memory, the processing device to: perform, using the four or more electrodes, an electrocardiogram (ECG) of the user.

2. The apparatus of claim 1, wherein to perform an ECG of the user, the processing device is to:

   measure a signal generated by each of the four or more electrodes when the housing cable is connected to the user; and
   calculate ECG data comprising a set of leads of the ECG based on the signal generated by each of the four or more electrodes.

3. The apparatus of claim 2, wherein the computing device further comprises:
   a transceiver to transmit the ECG data to a computing device.

4. The apparatus of any preceding claim, wherein the particular location contacted by each of the four or more electrodes correspond to a right arm, chest, left arm, and left leg of the user respectively.

5. The apparatus of any preceding claim, wherein each of the four or more electrodes are dry electrodes.

6. The apparatus of claim 5, wherein each of the four or more electrodes comprises a coupling mechanism to connect the electrode to a corresponding respective location of the user.

7. The apparatus of any of claims 1 to 5, wherein each of the four or more electrodes comprises an adhesive material to connect the electrode to a corresponding respective location of the user.

8. An apparatus comprising:

   a housing cable comprising a plurality of signal cables positioned therein;

four or more electrodes positioned along the housing cable to maintain conductive contact with a particular location of a user without any muscular activity of the user when the four or more electrodes are connected to the user; and

a computing device positioned along the housing cable and operatively coupled to each of the four or more electrodes via a respective signal cable of the plurality of signal cables, the computing device comprising:

a memory; and

a processing device operatively coupled to the memory, the processing device to:

perform, using the four or more electrodes, an electrocardiogram (ECG) of the user, the ECG comprising a set of ECG waveforms corresponding to leads formed by the four or more electrodes.

9. The apparatus of claim 8, wherein the housing cable comprises an elastic material.

10. The apparatus of claim 9, wherein the plurality of signal cables comprises one or more flexible printed circuits.

11. The apparatus of any of claims 8 to 10, wherein each of the four or more electrodes comprises a coupling mechanism to connect the electrode to a corresponding respective location of the user, each of the coupling mechanisms comprising one of: a clamp, a suction cup, a band, and a cuff brace.

12. The apparatus of any of claims 8 to 11, wherein the particular location contacted by each of the four or more electrodes correspond to a right arm, chest, left arm, and left leg of the user respectively.

13. A system comprising:

an apparatus comprising:

a housing cable comprising a plurality of signal cables positioned therein;

four or more electrodes positioned along the housing cable, each of the electrodes to contact a particular location of a user when the housing cable is connected to the user; and

a first computing device positioned along the housing cable and operatively coupled to each of the four or more electrodes via a respective signal cable, the computing device comprising:

a memory; and

a processing device operatively coupled to the memory, the processing device to:

measure a signal generated by each of the four or more electrodes;

determine first ECG data of the user based on the signal generated by each of the four or more electrodes, the first ECG data corresponding to leads measured by the processing device; and

transmit the first ECG data; and

a second computing device to:

receive the first ECG data; and

synthesize second ECG data of the user based on the first ECG data, the second ECG data corresponding to leads that were not measured by the processing device.

14. The system of claim 13, wherein the second computing device is further to:

determine one or more interpretations based on the first and second ECG data.

15. The system of claim 14, further comprising:

a mobile computing device to:

provide instructions to the user for connecting the apparatus to themselves such that each of the four or more electrodes is contacting the respective location of the user.

**FIG. 1**

EP 4 197 441 A1

EP 4 197 441 A1

FIG. 2

FIG. 3A

EP 4 197 441 A1

**FIG. 3B**

EP 4 197 441 A1

FIG. 3C

FIG. 3D

SIGNAL CABLES 325

COMPUTING UNIT 305

310A

310B

310C

310D

NETWORK 420

COMPUTING DEVICE 405

410

415

400

**FIG. 4A**

SIGNAL CABLES 325

COMPUTING UNIT 305

310A

310B

310C

310D

450

NETWORK 420

COMPUTING DEVICE 405

410

415

FIG. 4B

EP 4 197 441 A1

300

310B (V2 – dry electrode with suction cup)

305

320

310C (LA – dry electrode with band)

310A (RA – dry electrode with band)

310D (LL – dry electrode with band)

**FIG. 5A**

EP 4 197 441 A1

300

305

320

310A (RA – sticky conductive electrode)

310B (V2 – sticky conductive electrode)

310C (LA – sticky conductive electrode)

310D (LL – sticky conductive electrode)

*FIG. 5B*

EP 4 197 441 A1

600

505 including 305 and 310A (RA – dry electrode with band)

310B (V2 – dry electrode with suction cup)

310C (LA – dry electrode with band)

320

310D (LL – dry electrode with band)

*FIG. 5C*

600

310B (V2 – sticky conductive electrode)

320

310C (LA – sticky conductive electrode)

505 including 310A (RA – sticky conductive electrode)

310D (LL – sticky conductive electrode)

*FIG. 5D*

EP 4 197 441 A1

**FIG. 5E**

310

510B

510A

505

515 (underside of housing 510)

600

*FIG. 5F*

600

505

320

515 (underside of housing 510)

520

FIG. 6A

*FIG. 6B*

*FIG. 6C*

FIG. 6D

*FIG. 7A*

NORMAL R-WAVE PROGRESSION

SLOW R-WAVE PROGRESSION

predicted

Original

*FIG. 7B*

800

PROCESSING DEVICE 802

LEAD CONVERSION
AND
INTERPRETATION
INSTRUCTIONS
825

MAIN MEMORY 804

LEAD CONVERSION
AND
INTERPRETATION
INSTRUCTIONS
825

BUS 830

STATIC MEMORY
805

NETOWORK INTERFACE DEVICE
808

SIGNAL GENERATION DEVICE
815

NETWORK
820

DATA STORAGE DEVICE 818

MACHINE-READABLE
STORAGE MEDIUM 828

LEAD CONVERSION
AND
INTERPRETATION
INSTRUCTIONS
825

FIG. 8

# EUROPEAN SEARCH REPORT

Europäisches
Patentamt
European
Patent Office
Office européen
des brevets

**Application Number**

EP 22 20 5921

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/150398 A1 (INDIAN INST TECHNOLOGY BOMBAY) 8 August 2019 (2019-08-08) <br> * abstract * <br> * page 18, paragraph 3-5 * <br> * claim 1; figures 3,5 * | 1-5,7-15 | INV. <br> A61B5/271 <br> A61B5/252 <br> A61B5/254 <br> A61B5/256 <br> A61B5/259 |
| X | US 5 511 553 A (SEGALOWITZ JACOB [US]) 30 April 1996 (1996-04-30) <br> * abstract * <br> * column 25, line 46 – line 63 * <br> * column 35, line 12 – line 33 * <br> * claims 1,4,8; figures 1A,18,19,24-27,31 * | 1-5,8-15 | A61B5/00 <br> A61B5/266 <br> H01R3/00 <br> H01R4/00 |
| X | EP 1 645 224 A1 (MULA GALERA PILAR [ES]; VIDAL FORTUNY JORDI [ES]) 12 April 2006 (2006-04-12) <br> * abstract * <br> * paragraph [0032]; claims 1-6; figures 1-8 * <br> * the whole document * | 1-5,7-15 | |
| X | WO 2021/070195 A1 (PARALE GURUNATH PURUSHOTTAM [IN]) 15 April 2021 (2021-04-15) <br> * abstract * <br> * page 30, line 6 – line 13; claim 1; figure 4 * <br> * page 29, line 6 * | 1-15 | TECHNICAL FIELDS SEARCHED (IPC) <br> A61B <br> H01R |
| X | US 9 226 678 B1 (GHAFFARI DARIUSH [US]) 5 January 2016 (2016-01-05) <br> * abstract * <br> * claim 1; figures 2,3,5-7,11-13,28 * <br> * column 12, last paragraph – column 13, paragraph first * | 1-5,7-15 | |

-/--

The present search report has been drawn up for all claims

| Place of search | Date of completion of the search | Examiner |
|---|---|---|
| The Hague | 27 March 2023 | Delval, Christophe |

EPO FORM 1503 03.82 (P04C01)

page 1 of 2

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2003/216655 A1 (SCHRECK DAVID M [US]) 20 November 2003 (2003-11-20) * abstract * * the whole document * ----- | 1-15 | |
| A | US 6 891 379 B2 (DRAEGER MEDICAL SYSTEM INC [US]) 10 May 2005 (2005-05-10) * abstract * * the whole document * ----- | 1-15 | |

TECHNICAL FIELDS
SEARCHED      (IPC)

The present search report has been drawn up for all claims

| Place of search | Date of completion of the search | Examiner |
|---|---|---|
| The Hague | 27 March 2023 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 2 of 2**

## ANNEX TO THE EUROPEAN SEARCH REPORT
## ON EUROPEAN PATENT APPLICATION NO.

EP 22 20 5921

This annex lists the patent family members relating to the patent documents cited in the above-mentioned European search report.
The members are as contained in the European Patent Office EDP file on
The European Patent Office is in no way liable for these particulars which are merely given for the purpose of information.

27-03-2023

| Patent document cited in search report | | Publication date | Patent family member(s) | | Publication date |
|---|---|---|---|---|---|
| WO 2019150398 | A1 | 08-08-2019 | NONE | | |
| US 5511553 | A | 30-04-1996 | NONE | | |
| EP 1645224 | A1 | 12-04-2006 | EP | 1645224 A1 | 12-04-2006 |
| | | | ES | 2251310 A1 | 16-04-2006 |
| WO 2021070195 | A1 | 15-04-2021 | AU | 2020361759 A1 | 07-04-2022 |
| | | | US | 2022369983 A1 | 24-11-2022 |
| | | | WO | 2021070195 A1 | 15-04-2021 |
| US 9226678 | B1 | 05-01-2016 | NONE | | |
| US 2003216655 | A1 | 20-11-2003 | AU | 2003231148 A1 | 12-12-2003 |
| | | | CA | 2486168 A1 | 04-12-2003 |
| | | | CN | 1668242 A | 14-09-2005 |
| | | | CN | 101254095 A | 03-09-2008 |
| | | | EP | 1503666 A2 | 09-02-2005 |
| | | | ES | 2443964 T3 | 21-02-2014 |
| | | | HK | 1075596 A1 | 23-12-2005 |
| | | | JP | 4751971 B2 | 17-08-2011 |
| | | | JP | 2005525899 A | 02-09-2005 |
| | | | US | 2003216655 A1 | 20-11-2003 |
| | | | WO | 03099115 A2 | 04-12-2003 |
| | | | ZA | 200409773 B | 28-09-2005 |
| US 6891379 | B2 | 10-05-2005 | AU | 2003268395 A1 | 29-03-2004 |
| | | | CN | 1679119 A | 05-10-2005 |
| | | | EP | 1535291 A1 | 01-06-2005 |
| | | | JP | 5403846 B2 | 29-01-2014 |
| | | | JP | 5436383 B2 | 05-03-2014 |
| | | | JP | 2006514774 A | 11-05-2006 |
| | | | JP | 2011040404 A | 24-02-2011 |
| | | | US | 2004105245 A1 | 03-06-2004 |
| | | | WO | 2004023496 A1 | 18-03-2004 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 55341421